# EUROPEAN PATENT SPECIFICATION

(11) **EP 0 441 430 B1**
(45) Date of publication and mention of the grant of the patent: **23.03.1994**
(21) Application number: 91200187.2
(22) Date of filing: 30.01.1991
(51) Int. Cl.: B01J 23/42, B01J 23/62, C07C 5/32

(54) **Catalytic dehydrogenation of C2-C5 paraffins**
Katalytische Dehydrierung von C2-C5-Paraffinen
Déshydrogénation catalytique de paraffines C2-C5

(30) Priority: 07.02.1990 IT 1928390
(43) Date of publication of application: 14.08.1991
(73) Proprietor: SNAMPROGETTI S.p.A., I-20121 Milan (IT)
(72) Inventor: Iezzi, Rodolfo, I-20097 San Donato Milanese, Milan (IT); Buonomo, Franco, I-20097 San Donato Milanese, Milan (IT); Sanfilippo, Domenico, I-20067 Paullo, Milan (IT)
(74) Representative: Appoloni, Romano

(56) References cited:
- EP-A- 0 351 066
- BE-A- 753 072
- GB-A- 2 071 071
- US-A- 4 413 152

## Description

This invention relates to a process for catalytically dehydrogenating C₂-C₅ paraffins to their corresponding olefins.

The evergrowing demand for olefins to be used for producing a number of chemicals, such as high-octane gasolines, synthetic elastomers, detergents, plastics materials, ion-exchange resins, pharmaceuticals and others, causes the paraffin dehydrogenation processes to play a significant commercial role, with particular respect to the dehydrogenation of light paraffins directed to the production of corresponding olefins to be used for preparing gasolines, alkylating aromatics, conversion into aromatics, production of isobutene for preparing MTBE (methyl tert.butyl ether) and other chemicals.

To be commercially interesting, such processes must have high paraffin conversions and a high selectivity, the secondary reactions being minimized.

The prior art has proposed a number of approaches, as well as a number of catalyst systems.

US-A-4 438 288 has suggested noble metals supported on substrates such as gamma-alumina, silica, magnesium oxides, supplemented by alkali metals or alkaline-earth metals: such catalytic systems have low activity and selectivity and a short active life.

Improvements were attempted by adopting catalyst system comprising platinum associated with tin, supported on gamma-, eta-, delta- and theta-alumina, such as in US-A-3 909 451 and US-A-3 998 900.

US-A-4 413 152 has suggested zerovalent metals of the VIII Group dispersed on titanated alumina, but as hydrogenation catalysts to be used in reducing aqueous soution of carbohydrates.

Finally, EP-A-0 351 066 Al has proposed an alkali-free catalyst system for dehydrogenating C₂-C₁₀ paraffins, based on a metal of the platinum group and tin.

All of these processes and catalyst systems of the prior art are unsatisfactory in that they exhibit a poor activity, a short active life, and, above all, an inadequate selectivity.

In order to overcome the defects outlined above, this invention proposes a process for selectively dehydrogenating C₂-C₅ paraffins to their corresponding olefins, comprising the step of reacting a gaseous stream of a C₂-C₅ paraffin in a reactor containing a supported catalytic composition consisting of:
- platinum, in an amount of from 0,01% by weight to 3% by weight;
- tin, in an amount of from 0,6% by weight to 1,5% by weight, and
- a supporting substrate selected from titanated alumina, titanated silica, and/or titanium silicalite, the Ti content in said supporting substrate being of from 0,05% by weight to 3% by weight,

at a temperature of from 500°C to 700°C, under a pressure of from 0,98066 bar to 1,96132 bar (1 kg/cm² to 2 kg/cm²) and at a GSHV (gas hourly space velocity) of from 100 h⁻¹ to 10000 h⁻¹.

Titanium-silicalite is a synthetic zeolite in whose crystal lattice silicon is partially replaced by titanium.

Said zeolite and the method to prepare it were disclosed in GB-A-2 071 071.

Some examples are now given to better illustrate the invention.

### Example 1

A sample of titanium-silicalite with a particle-size smaller than 30 µm, prepared according to the process described in Example 1 of GB-A-2 071 071 is impregnated with a solution acidified by means of HCl, containing hexachloroplatinic acid and stannous chloride dihydrate, by using the following procedure:

1,16 g of SnCl₂.2H₂O is dissolved in 8,6 cm³ of concentrated hydrochloric acid and the resulting solution is added to 4,07 g (2,5 cm³) of an aqueous solution of hexachtoroplatinic acid (containing 25% by weight of Pt).

The volume of the solution is adjusted to a total value of 60 cm³ with water, and the resulting solution is slowly added to 100 g of titanium silicalite.

When impregnation is complete, the resulting material is concentrated to dryness at a temperature of round 120°C, over a time of 4 hours.

The dried material is calcined in a stream of air flowing at a GHSV (Gas Hourly Space Velocity) of 500 h⁻¹, over a time of 2 hours.

The calcined product is subsequently reduced in a stream of H₂/N₂ (molar ratio 1), at the temperature of 600°C and flowing at a GHSV of 500 h⁻¹, over a time of 2 hours.

A catalyst is eventually obtained, which has the chemical composition: 1% by weight of Pt, 0.6% by weight of Sn, 0.02% by weight of Cl⁻, 98.38% by weight of titanium-silicalite (2% by weight of Ti).

The catalyst is tested in the dehydrogenation of propane is a fluidized-bed reactor by operating at the temperature of 590°C, under a pressure of 0,98066 bar (1 kg/cm²) and with a gas hourly space velocity (GHSV) of 400 h⁻¹ referred to a catalyst bed obtained by diluting the catalyst in α-alumina with a ratio of catalyst to α-alumina of 0,06 by weight.

The results obtained are reported in Tables 1 and 2.

### Example 2 - Comparative Example

A sample of 100 g of silicalite, with a granulometry smaller than 30 µm, with the average size of the crystals being of from 20 µm to 26 µm, is impregnated with SnCl₂.2H₂O and hexachloroplatinic acid, in solution acidified with hydrochloric acid, with such amounts being used, to obtain an end catalyst having the following chemical composition: 1% by weight of Pt, 0,6% by weight of Sn, 0,01% by weight of Cl⁻, 98,39% by weight of silicalite.

The procedures of impregnation, drying, calcination, and reduction are as disclosed in Example 1. The catalyst is eventually tested according to the procedure reported in Example 1, in the reaction of propane dehydrogenation.

The results obtained are shown in Table 1.

### Example 3

A sample of 100 g of microspheroidal alumina prepared according to the prior art with a particle-size smaller than 30 µm, a surface area of 250 m²/g and a total porosity of 0,8 cm³/g is impregnated with an alcoholic solution obtained by dissolving 7,2 of tetraethyl orthotitanate in 70 cm³ of ethyl alcohol, according to the following procedure.

100 g of alumina are charged to a quartz reactor installed inside a heating furnace.

From the bottom end of said quartz reactor, through the distributor, nitrogen is fed with such a flow rate as to have a linear speed inside the interior of the reactor, of 2cm/s, which causes the material to be fluidized. From the top end of the reactor an alcoholic solution of Ti(OC₂H₅)₄ -- obtained by dissolving 7,2 g of tetraethyl orthotitanate in 70 cm³ of ethyl alcohol -- is added dropwise, with the material being constantly kept at room temperature. When addition is complete, the heating of the material is started, and the material is gradually heated up to 550°C. When this temperature is reached, nitrogen flow is discontinued and the material is calcined in an air flow over 2 hours.

The product obtained after calcination at 550°C, containing 2,5% of TiO₂ by weight, is impregnated with a solution acidified with HCl, containing suitable amounts of SnCl₂.2H₂O and H₂PtCl₆, such as to obtain an end product containing: 1% by weight of Pt,0,6% by weight of Sn, 2,45 by weight of TiO₂, 0,02% by weight of Cl-, 95,93% by weight of Al₂O₃.

The procedures of impregnation and activation are the same as already cited in the preceding examples.

The catalyst was finally tested in the dehydrogenation of propane under the conditions of Example 1. The results obtained are reported in Table 1.

### Example 4 - Comparative Example

A sample of microspheroidal alumina, prepared according to the prior art, with a particle-size smaller than 30 µm, a surface area of 250 m²/g and a total porosity of 0,8 cm³/g is impregnated with a solution acidified with hydrochloric acid, containing hexachloroplatinic acid and stannous chloride dihydrate, by the following procedure:

1,16 g of SnCl₂.2H₂O is dissolved in 8,6 cm³ of concentrated hydrochloric acid and the resulting solution is added to 4,07 g (2,5 cm³) of an aqueous solution of hexachloroplatinic acid (at 25% by weight of Pt)

The volume of the solution is adjusted to a total value of 80 cm³ with water, and the resulting solution is slowly added to 100 g of alumina.

At the end of the impregnation, the material is concentrated to dryness, calcined and reduced under the same conditions as already described in Example 1.

A catalyst with the following chemical composition: 1% by weight of Pt, 0,6% by weight of Sn, 0,02% by weight of Cl⁻, 98,38% by weight of Al₂O₃ is obtained.

The catalyst is tested in the dehydrogenation of propane on a fluidized-bed catalyst, by following the procedure of Example 1.

The results are shown in Tables 1 and 2.

### Example 5 (Comparative Example)

A sample of the same alumina as used in Example 4 is admixed with silica by using esters of silicic acid, according to the following procedure.

100 g of alumina is charged to a quartz reactor installed inside a heating furnace.

From the bottom end of the quartz reactor, through the distributor, nitrogen is fed with such a flow rate as to have a linear speed inside the interior of the reactor, of 2 cm/s, which causes the material to be fluidized. From the top an alcoholic solution of Si(OC₂H₅)₄ -- obtained by dissolving 34 g of tetraethyl orthosilicate in 43 cm³ of ethyl alcohol -- is added dropwise, with the material being constantly kept at room temperature. When addition is complete, the heating of the material is started, and the material is gradually heated up to 550°C. When this temperature is reached, nitrogen flow is discontinued and the material is calcined in an air flow over 2 hours.

The resulting product has the following chemical composition: 11,5% by weight of SiO₂, 88,5% by weight of Al₂O₃, with a surface area of 245 m²/g and a total porosity of 0,7 cm³/g.

100 g of this material is impregnated with the same amounts as used in Example 1, of SnCl₂.2H₂O and hexachloroplatinic acid, by the same procedure as already known for catalyst preparation.

The catalyst is tested in the dehydrogenation of propane according to the modalities described in Example 1.

The results obtained are reported in table 1.

### Example 6

100 g of silica with a particle-size smaller than 30 µm a surface area of 250 m²/g and a total porosity of 0,6 cm³ is impregnated with an alcoholic solution obtained by dissolving 7,2 g of tetraethyl orthotitanate in 53 cm³ of ethyl alcohol, using the same procedure as described in Example 5. The product obtained after calcination at 550°C, containing 2,5% of TiO₂, is impregnated with a solution acidified with hydrochloric acid, and containing such suitable amounts of SnCl₂.2H₂O and H₂PtCl₆ as to obtain eventually a product containing 1% by weight of Pt, 0,6% by weight of Sn, 2,45% by weight of TiO₂ and 95,93% by weight of Al₂O₃ and 0,01% by weight of Cl⁻.

The procedures followed for the impregnation and the activation are the same as already cited in the preceding examples.

The catalyst is eventually tested in propane dehydrogenation, under the conditions set forth in Example 1.

The results obtained are reported in Table 1.

### Example 7 - Comparative Example

100 g of silica with a particle-size smaller than 30 µm, a surface area of 250 m²/g and a total porosity of 0,6 cm³ is impregnated with an aqueous solution acidified with hydrochloric acid, containing SnCl₂.2H₂O and hexachloroplatinic acid (same amounts as of Example 1), then impregnated silica is dried, calcined and reduced by following the same procedure also as described in Example 1.

At the end a catalyst is obtained, having the following chemical composition: 1% by weight Pt, 0,6% by weight Sn, 0,02% by weight Cl⁻, 98,38% by weight SiO₂, a surface area of 245 m²/g, and a total porosity of 0.55 cm³/g.

The catalyst was finally tested in propane dehydrogenation, under the same conditions as described in Example 1.

The results obtained are shown in Table 1.

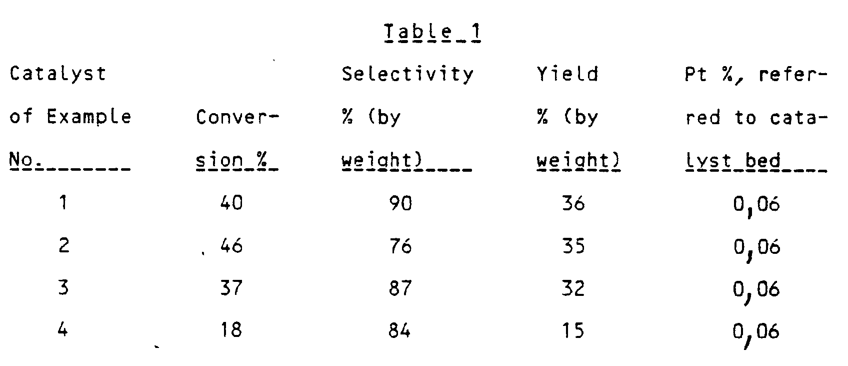

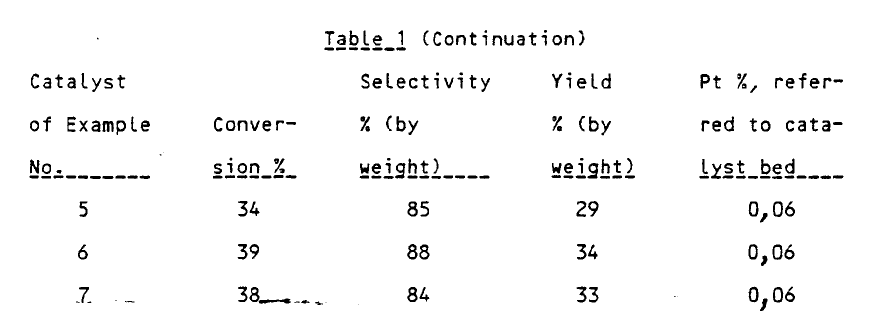

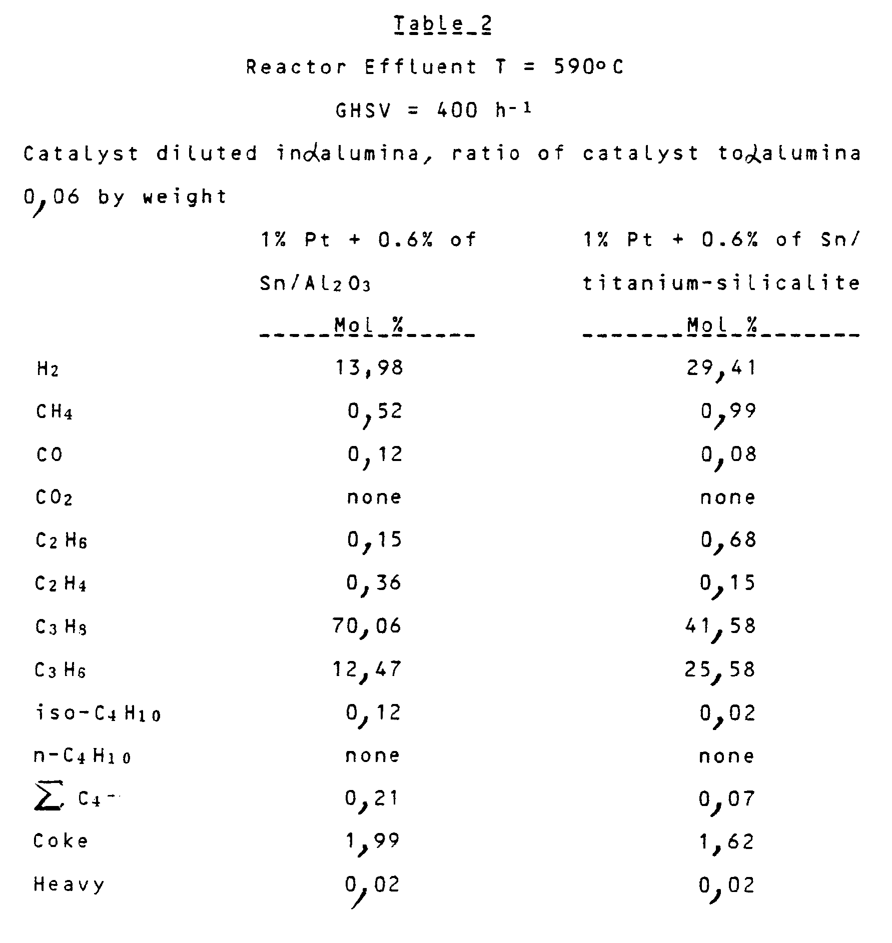

## Claims

1. Process for selectively dehydrogenating C₂-C₅ paraffins to their corresponding olefins, comprising the step of reacting a gaseous stream of a C₂-C₅ paraffin in a reactor containing a supported catalytic composition consisting of:
- platinum, in an amount of from 0,01% by weight to 3% by weight;
- tin, in an amount of from 0,6% by weight to 1,5% by weight, and
- a supporting substrate selected from titanated alumina, titanated silica, and/or titanium silicalite, the Ti content in said supporting substrate being of from 0,05% by weight to 3% by weight,
at a temperature of from 500°C to 700°C, under a pressure of from 0,98066 bar to 1,96132 bar (1 kg/cm² to 2 kg/cm²) and at a GSHV (gas hourly space velocity) of from 100 h⁻¹ to 10000 h⁻¹.

2. Process, according to Claim 1, wherein the Ti content in the supporting substrate is of from 1% by weight to 2% by weight.

3. Process according to Claim 1, wherein the reactor is a fluidized-bed reactor.

4. Process according to Claim 1, wherein said paraffin is propane.

## Patentansprüche

1. Verfahren zur selektiven Dehydrierung von C₂-C₅-Paraffinen zu ihren entsprechenden Olefinen, umfassend den Schritt der Umsetzung eines C₂-C₅-Paraffin-Gasstroms in einem Reaktor, enthaltend eine auf einem Träger aufgebrachte katalytische Zusammensetzung bestehend aus:
- Platin in einer Menge von 0,01 Gew.-% bis 3 Gew.-%,
- Zinn in einer Menge von 0,6 Gew.-% bis 1,5 Gew.-%, und
- einem Trägersubstrat, gewählt aus titanhaltigem Aluminiumoxid, titanhaltigem Siliciumoxid und/oder Titansilicalit, wobei der Ti-Gehalt in dem Trägersubstrat 0,05 Gew.-% bis 3 Gew.-% beträgt,
bei einer Temperatur von 500°C bis 700°C, einem Druck von 0,98066 bar bis 1,96132 bar (1 kg/cm² bis 2 kg/cm²) und einer Gas-Belastung des Katalysators pro Stunde (GHSV) von 100 h⁻¹ bis 10 000 h⁻¹,

2. Verfahren nach Anspruch 1, wobei der Ti-Gehalt im Trägersubstrat 1 Gew.-% bis 2 Gew.-% beträgt.

3. Verfahren nach Anspruch 1, wobei der Reaktor ein Fließbett-Reaktor ist.

4. Verfahren nach Anspruch 1, wobei das Paraffin Propan ist.

## Revendications

1. Procédé d'hydrogénation sélective de paraffines en C₂-C₅ en les oléfines correspondantes, comprenant l'étape consistant à faire réagir un courant gazeux d'une paraffine en C₂-C₅ dans un réacteur contenant une composition de catalyseur supporté, constituée par :
- du platine, à raison de 0,01 à 3 % en poids,
- de l'étain, à raison de 0,6 à 1,5 % en poids, et
- un substrat servant de support, choisi parmi une alumine traitée avec un titanate, une silice traitée avec un titanate, et/ou une silicalite au titane, la teneur en titane dudit substrat servant de support valant de 0,05 à 3 % en poids,
à une température valant de 500°C à 700°C, sous une pression valant de 0,98066 à 1,96132 bar (1 à 2 kg/cm²) et à une VSHG (vitesse spatiale horaire des gaz) valant de 100 a 10000 h⁻¹.

2. Procédé conforme à la revendication 1, dans lequel la teneur en titane dudit substrat servant de support vaut de 1 à 2 % en poids.

3. Procédé conforme à la revendication 1, dans lequel le réacteur est un réacteur à lit fluidisé.

4. Procédé conforme à la revendication 1, dans lequel ladite paraffine est du propane.
